# EUROPÄISCHE PATENTSCHRIFT

(11) **EP 1 981 966 B1**
(45) Veröffentlichungstag und Bekanntmachung des Hinweises auf die Patenterteilung: **05.01.2011**
(21) Anmeldenummer: 07703330.6
(22) Anmeldetag: 07.02.2007
(51) Int. Cl.: C12N 5/07, G01N 33/50

(54) **VERWENDUNG VON HUMANEN HEPATOZYTEN UND IN-VITRO-VERFAHREN ZUR ERMITTLUNG DER LEBERFUNKTION UND LEBERREGENERATION**
USE OF HUMAN HEPATOCYTES AND IN-VITRO-PROCESS FOR DETERMINING LIVER FUNCTION AND LIVER REGENERATION
UTILISATION D'HÉPATOCYTES HUMAINS ET PROCÉDÉ IN VITRO POUR DÉTERMINER LA FONCTION HÉPATIQUE ET POUR LA RÉGÉNÉRATION DU FOIE

(30) Priorität: 07.02.2006 DE 102006005526
(43) Veröffentlichungstag der Anmeldung: 22.10.2008
(73) Patentinhaber: Sauer, Martin, 18055 Rostock (DE)
(72) Erfinder: Sauer, Martin, 18055 Rostock (DE)
(74) Vertreter: Garrels, Sabine
(86) Internationale Anmeldenummer: PCT/EP2007/001047
(87) Internationale Veröffentlichungsnummer: WO 2007/090633

(56) Entgegenhaltungen:
- SKELLY MAEVE M ET AL: "Findings on liver biopsy to investigate abnormal liver function tests in the absence of diagnostic serology." JOURNAL OF HEPATOLOGY, Bd. 35, Nr. 2, August 2001 (2001-08), Seiten 195-199, XP002432459 ISSN: 0168-8278
- KIMURA S ET AL: "Indocyanine green elimination rate detects hepatocellular dysfunction early in septic shock and correlates with survival." CRITICAL CARE MEDICINE JUN 2001, Bd. 29, Nr. 6, Juni 2001 (2001-06), Seiten 1159-1163, XP002432460 ISSN: 0090-3493 in der Anmeldung erwähnt

## Beschreibung

Die vorliegende Erfindung betrifft die Verwendung von humanen Hepatozyten zur in-vitro-Ermittlung der Leberfunktion und Leberregeneration bei einem Probanden, insbesondere bei einem Patienten, sowie die Verwendung dieser humanen Hepatozyten als Prognoseparameter. Ferner betrifft die vorliegende Erfindung ein in-vitro-Verfahren zur Ermittlung der Leberfunktion und Leberregeneration bei einem Probanden, insbesondere bei einem Patienten, unter Verwendung von humanen Hepatozyten.

### Stand der Technik

Die Krankheitsbilder "Systemic Infiammatory Response Syndrom" (SIRS) mit Multiorganversagen, schwere Sepsis und septischer Schock stehen häufig im Zusammenhang mit der Entwicklung eines reversiblen Leberschadens, wodurch es zu einer Einschränkung der Leberfunktionen kommen kann. Eine regelmäßige Medikamenteneinnahme, ein hoher Alkoholgenuss oder auch Infektionen können die Leber in ihrer Funktion ebenso erheblich beeinträchtigen. Die oben genannten Krankheitsbilder können in seltenen Fällen zu einem irreversiblen Leberversagen führen.

Eine eingeschränkte bzw. gestörte Leberfunktion kann z.B. eine Störung der Gehirnfunktion, wie die hepatische Enzephalopathie, zur Folge haben. Verursacht wird diese durch die verminderte Metabolisierung von Eiweissabbauprodukten in der Leber. Symptomatisch macht sich eine eingeschränkte Leberfunktion z.B. durch eine geringere Leistungsfähigkeit, Müdigkeit, Schläfrigkeit oder selten auch Druckgefühl im Oberbauch bemerkbar.

Im septischen Schock entwickeln schließlich ungefähr 22 % aller Patienten ein Leberversagen (1). Als Leberversagen oder Leberinsuffizienz wird das Erlöschen der Leberfunktion bis hin zum hepatischen Koma bezeichnet. Das Leberversagen ist gekennzeichnet durch das Auftreten von Gelbsucht (Ikterus), hepatischer Enzephalopathie, hepatorenalem Syndrom, Blutgerinnungsstörungen, Hypalbuminämie mit Aszites, Thrombozytopenie oder endokrinen Störungen.

Harbrecht und Mitarbeiter konnten in einer Studie nachweisen, dass ein Leberversagen, welches sich im Verlauf eines Krankenhausaufenthaltes entwickelt, die Krankenhausliegezeit verlängert und die Letalität erheblich erhöht (2). Die Gründe dafür sind noch nicht bis ins Einzelne geklärt. Bisherige Erklärungsmodelle gehen von einer Schädigung der Leber aus, die aus einer Einschränkung der Splanchnikusperfusion, aus einer Endotheldysfunktion und einer direkten Schädigung durch bakterielle oder andere, wie z.B. endogene Toxine herrührt (3-6).

Schließlich ist anzumerken, dass beim akuten Leberversagen, das nicht im Rahmen von SIRS oder Sepsis auftritt, verstärkt humorale und zelluläre Inflammationsmarker nachzuweisen sind (7).

Sepsis, SIRS mit Multiorganversagen und Leberversagen stehen weltweit mit an der Spitze der Todesursachen. In den USA erkranken pro Jahr etwa 500.000 Patienten an einer Sepsis mit einer durchschnittlichen Letalität von 21 % (8). In Deutschland sterben jährlich 70.000 Patienten an dieser Erkrankung und deren Komplikationen. Ca. 20.000 Patienten pro Jahr sterben in Deutschland an einer Lebererkrankung. Bei Auftreten eines fulminanten Leberversagens bleibt letztendlich nur die Lebertransplantation.

Insgesamt ist festzustellen, dass die Ermittlung bzw. Diagnose einer eingeschränkten bzw. gestörten Leberfunktion bei einem Probanden zum frühest möglichen Zeitpunkt von größter Bedeutung ist, da somit entsprechend ein akutes Leberversagen frühzeitig behandelt oder diesem frühzeitig vorgebeugt werden kann.

Eine eingeschränkte bzw. gestörte Leberfunktion kann derzeit durch die Messung verschiedener leberspezifischer und unspezifischer Blutwerte diagnostiziert werden. So kann durch die Messung der Leberenzyme Glutamat-Pyruvat-Transaminase (GPT), GlutamatOxalacetat-Transaminase (GOT) und Gamma^Glutamyl-Transferase (Gamma-GT) eine Leberschädigung nachgewiesen werden. Eine Störung der exkretorischen Funktion und Entgiftungsfunktion der Leber hat zum Beispiel erhöhte Werte von Billirubin, Gamma-GT, Ammoniak und alkalischer Phosphatase (AP) zur Folge. Bei eingeschränkter Syntheseleistung der Leber kann sich auch der Gehalt an Proteinen im Plasma, wie z.B. dem Albumin und vieler Gerinnungsfaktoren, vermindern.

Die derzeit verwendeten diagnostischen Verfahren haben jedoch zum Nachteil, dass viele der genannten Blutwerte erst bei fortgeschrittenem Leberversagen deutlich im pathologischen Bereich sind und somit zur Früherkennung eines Leberversagens ungeeignet sind.

Zur Ermittlung der Leberfunktion werden ferner Verfahren herangezogen, die z.B. die Splanchnikusperfusion ermitteln. Dies erfolgt z.B. mit dem LiMON-System (PULSION Medical Systems AG, Deutschland).

Das LiMON-System zur Ermittlung der Splanchnikusperfusion und Leberfunktion befindet sich derzeit in der klinischen Erprobung (3-4). Bei diesem Verfahren gestaltet es sich aber aus technischen Gründen bei insbesondere hoch septischen Patienten sehr schwierig, eine korrekte Messung mit diesem System durchzuführen.

Zusammenfassend lässt sich sagen, dass die gegenwärtig gebräuchlichen Verfahren nur relativ unspezifische Aussagen hinsichtlich der Leberfunktion bei einem Patienten liefern.

Bekannt sind aus US Patent 5,290,684 A humane Hepatozyten einer HepG2/C3A-Zellinie, welche hier lediglich therapeutisch angewendet werden. Diese können kommerziell erworben werden.

### Darstellung der Erfindung

Aufgabe der Erfindung ist es somit einen neuen Zytotoxititätstest auf der Basis humaner Hepatozyten bereitzustellen, der die direkte Schädigung der Leber und ihrer Regeneration bei einem Probanden erfasst.

Erfindungsgemäß wird zur Lösung dieser Aufgabe ein neues in-vitro-Verfahren unter Verwendung von humanen Hepatozyten bzw. eine neue Verwendung von humanen Hepatozyten bereitgestellt, so dass die Leberfunktion eines Probanden, insbesondere bei einem Patienten, der an dem Systemic Inflammatory Response Syndrome (SIRS), Sepsis, Leberversagen oder Multiorganversagen erkrankt ist, auf zellulärer Ebene ermittelt werden kann.

Vergleichbare Untersuchungen auf zellulärer Ebene zur Ermittlung der Leberfunktion bei Probanden, insbesondere bei einem Patienten mit eingeschränkter bzw. gestörter Leberfunktion, wie mit dem erfindungsgemäßen in-vitro-Verfahren bereitgestellt wird, sind bisher nicht bekannt. Insbesondere ist ein Zytotoxititätstest auf der Basis humaner Hepatozyten, der die direkte Schädigung der Leber bei einem Patienten mit den Krankheitsbildern SIRS mit Multiorganversagen, schwere Sepsis, septischer Schock und Leberversagen auf zellulärer Ebene erfasst, ist nicht bekannt.

Das erfindungsgemäße in-vitro-Verfahren bzw. die erfindungsgemäße Verwendung von humanen Hepatozyten zur in-vitro-Ermittlung der Leberfunktion auf zellulärer Ebene hat zum Vorteil, dass Aussagen über die Leberfunktion eines Probanden, insbesondere eines Patienten, wesentlich genauer und frühzeitiger im Vergleich zu den derzeit verfügbare Verfahren getroffen werden können.

Es sei darauf hingewiesen, dass der Begriff "Proband" im Folgenden sowohl einen "gesunden Probanden" als auch einen "kranken Probanden" im Sinne eines Patienten umfasst.

Das erfindungsgemäße in-vitro-Verfahren bzw. die erfindungsgemäße Verwendung von humanen Hepatozyten zur in-vitro-Ermittlung der Leberfunktion beruht auf dem Prinzip, dass jede Substanz, in Abhängigkeit von ihrer Konzentration und Kombination mit anderen Substanzen, auf humane Zellen toxische Einflüsse haben kann. Mit dem erfindungsgemäßen Verfahren bzw. Verwendung wird damit ein Zytotoxizitätstest bereitgestellt, wobei die endogene und exogene (z.B. durch Medikamente hervorgerufene) Zytotoxizität erfasst wird.

Erfindungsgemäß werden zur Ermittlung der Leberfunktion eines Probanden humane Hepatozyten mit Probandenplasma inkubiert. Als Parameter für die Leberfunktion eines Probanden ist die Zellschädigung der humanen Hepatozyten nach Inkubation mit dem Probandenplasma anzusehen. Insbesondere kann, nach Inkubation der humanen Hepatozyten mit Probandenplasma, die Leberfunktion und Leberregeneration eines Probanden mittels Messung bestimmter Parameter, wie zum Beispiel Vitalität, Funktionalität und Aktivierungsgrad der humanen Hepatozyten, abgeleitet werden.

Das erfindungsgemässe in-vitro-Verfahren zur Ermittlung der Leberfunktion wird auf der Basis von humanen Hepatozyten, die bevorzugt aus den humanen Leberzelllinien C3A, HLE, HepG2, HuH7, Hep3B, PLC/PRF/5 ausgewählt sind, durchgeführt. Die entsprechenden Zelllinien wurden von der American Type Culture Collection (ATCC) erworben, und erfüllen damit allgemeingültige Qualitätsstandards. Der Zytoxizitätstest enthält zwei Teile eines Testkits: Der erste Teil umfasst mit Humanhepatozyten belegte Mikroplatten (24-Well-Platten). Der zweite Teil umfasst gebrauchsfertige Lösungen von für den Test notwendigen Reagenzien.

Zur Ermittlung der Leberfunktion werden die humanen Hepatozyten mit Probandenplasma inkubiert. Das Probandenplasma wird praktisch als zellfreier Überstand nach Zentrifugation von antikoaguliertem Blut gewonnen. Die Gerinnung der Blutprobe wird durch Zusatz von Antikoagulanzien, wie zum Beispiel Heparin bzw. Heparinoide oder Substanzen, die Calcium-Ionen binden, wie Citrat, EDTA, Fluoride oder Oxalat, gehemmt. Die Gewinnung von Plasma und die dazu notwendigen Antikoagulanzien-Konzentration und Zusammensetzung sind in einer internationalen Norm standardisiert und sollte somit dem Fachmann bekannt sein.

Das erfindungsgemäße in-vitro-Verfahren bzw. die erfindungsgemäße Verwendung von humanen Hepatozyten zur in-vitro-Ermittlung der Leberfunktion beruht auf dem Prinzip, dass durch eine eingeschränkte bzw. gestörte Leberfunktion bestimmte Substanzen und Stoffwechselmetabolite, wie endogene und exogene Toxine, im Plasma eines Probanden kumulieren. Diese Substanzen können schließlich einen toxischen Einfluss auf die humanen Hepatozyten haben. Durch Bestimmung bestimmter Vitalitäts-, Funktions- und Aktivierungsparameter nach Inkubation der humanen Hepatozyten mit dem Probandenplasma ist es somit möglich die Leberfunktion eines Probanden abzuleiten.

So kann nach Inkubation der humanen Hepatozyten mit Probandenplasma die Leberfunktion des Probanden mittels Messung der Aktivität von Cytochrom P450 Isoenzym 1A2 (CYP 1A2) der humanen Hepatozyten abgeleitet werde.

Die Cytochrom P450-Superfamilie besteht aus mikrosomalen Hämoproteinen, die für den oxidativen, peroxidativen und reduktiven Stoffwechsel einer Reihe von endogenen und exogenen Stoffen eine zentrale Bedeutung haben.

Eine weitere Einteilung der Cytochrome erfolgt in Enzymfamilien, die eine Übereinstimmung der Proteinsequenz von mindestens 40 % zeigen, und in Subfamilien, deren Übereinstimmung bei über 55 % liegt.

Cytochrom P4501A2 (CYPI A2) konnte beim Menschen bis jetzt ausschließlich in der Leber nachgewiesen werden und macht dort etwa 15 % des gesamten Cytochrom-Gehaltes aus. Seine Expression ist abhängig von verschiedenen Faktoren, u. a. von Geschlecht, Rasse, genetischen Polymorphismen und der Exposition gegenüber Induktoren, so dass es große interindividuelle Unterschiede gibt.

Die Funktion von CYP 1A2 liegt hauptsächlich im Fremdstoffmetabolismus (Umwelt- und Nahrungsgifte, Arzneimittel).

Die Aktivität von Cytochrom P450 1A2, einem an der Biotransformation verschiedener Arzneimittel beteiligten Enzym, ist bei HepG2/C3A-Zellen zwar gering, kann jedoch durch Induktion mit Methylcholanthren auf ein über 40faches des Ausgangswertes gesteigert werden (12).

In einer anderen Ausführungsform, kann nach Inkubation der humanen Hepatozyten mit Probandenplasma die Leberfunktion des Probanden mittels Messung der Aktivität der humanen Hepatozyten Mikroalbumin und andere Proteine zu synthetisieren, abgeleitet werden.

Albumine sind gut wasserlösliche globuläre Proteine. Sie bestehen aus etwa 580 Aminosäuren und haben ein durchschnittliches Molekulargewicht von 66 000 Dalton. Albumine bilden etwa 60 % des Gesamteiweisses im Blutplasma und dienen vor allem zur Regelung des intravasalen onkotischen Drucks und als Transportproteine für wasserunlösliche Stoffe wie z. B. Fettsäuren, Hormone, Spurenelemente und auch verschiedene Arzneimittel. Substanzen mit toxischer Wirkung können die Transportfähigkeit der Albumine durch die Bindung an diese verringern.

Außerdem stellen Albumine eine Aminosäure-Reserve für den Körper dar und tragen zur Pufferkapazität des Blutes bei.

Die Albuminsynthese findet ausschließlich in der Leber statt und repräsentiert etwa 10 % der gesamten Synthesekapazität für Proteine in der Leber. Aus diesem Grund gilt die Aminosäuresynthese als wichtiger Parameter für die Funktion der Leber. Ein Erwachsener bildet täglich circa 120-200 mg Albumin / kg Körpergewicht.

In einer anderen Ausführungsform, kann nach Inkubation der humanen Hepatozyten mit Probandenplasma die Leberfunktion des Probanden mittels Messung der Freisetzung von Lactat-Dehydrogenase aus den humanen Hepatozyten abgeleitet werden. Die Lactat-Dehydrogenase (LDH) ist ein Zellenzym, das eine große Bedeutung für den Intermediärstoffwechsel der Zelle besitzt, da das Enzym bei der Glykolyse mitwirkt. Es katalysiert die Reaktion von Pyruvat zu Lactat, als Coenzym dient dabei NADH.

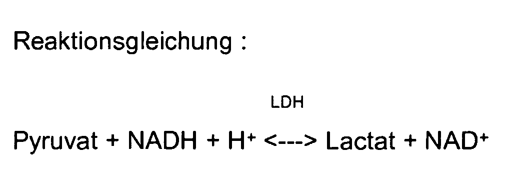

LDH ist in vielen verschiedenen Geweben nachweisbar, u.a. in der Leber, der Herz- und Skelettmuskulatur, den Erythrozyten, dem lymphatischen und hämatopoetischen Gewebe sowie in der Niere und in Malignomen.

Es gibt fünf verschiedene Isoenzyme, die eine unterschiedliche Organverteilung besitzen. Die Plasma-Halbwertzeit der Lactat-Dehydrogenase liegt bei 10-120 Stunden. Der Normalwert für die LDH-Aktivität im Blutserum liegt bei 120-240 U/I und resultiert aus der täglichen Zellmauserung.

Eine Erhöhung dieser Aktivität findet man bei einer Schädigung der Herkunftszellen, wobei es durch Permeabilitätsstörungen der Zellmembran oder Lyse der Zellen zum Austritt des Enzyms kommen kann. Dies kann auch bei verstärkter Zellmauserung und bei regenerativen Prozessen der Fall sein.

In einer anderen Ausführungsform, kann nach Inkubation der humanen Hepatozyten mit Probandenplasma die Leberfunktion des Probanden mittels Messung der Adhärenz der vitalen humanen Hepatozyten abgeleitet werden.

In einer anderen Ausführungsform, kann nach Inkubation der humanen Hepatozyten mit Probandenplasma die Leberfunktion des Probanden mittels Messung der Esteraseaktivität der vitalen humanen Hepatozyten abgeleitet werden.

In einer anderen Ausführungsform, kann nach Inkubation der humanen Hepatozyten mit Probandenplasma die Leberfunktion des Probanden mittels Messung der Anzahl toter und der Anzahl lebender humaner Hepatozyten abgeleitet werden.

In einer anderen Ausführungsform, kann nach Inkubation der humanen Hepatozyten mit Probandenplasma die Leberfunktion des Probanden mittels Messung der Aktivität mitochondrialer Dehydrogenasen der vitalen humanen Hepatozyten abgeleitet werden.

Das erfindungsgemäße in-vitro-Verfahren bzw. die Verwendung von humanen Hepatozyten eignet sich zur Ermittlung der Leberfunktion bei Probanden, die an dem Systemic Inflammatory Response Syndrome (SIRS), Sepsis, septischen Schock, Leberversagen oder Multiorganversagen erkrankt sind.

Außerdem eignet sich das erfindungsgemäße in-vitro-Verfahren bzw. die Verwendung von humanen Hepatozyten zur Früherkennung eines Leberversagens, zur Überprüfung von Therapien, des Behandlungsverlaufes und der Regeneration der Leberfunktion.

Ferner eignet sich das erfindungsgemäße in-vitro-Verfahren bzw. die Verwendung von humanen Hepatozyten als Prognoseparameter, mit welchem es möglich ist, schon frühzeitig einen Probanden auf eine Lebertransplantation vorzubereiten, wenn er z.B. eine schlechte Prognose hinsichtlich seiner Lebererkrankung hat. Bei lebertransplantierten Probanden trägt das erfindungsgemäße Verfahren bzw. Verwendung dazu bei, genauere Aussagen über die Funktionsfähigkeit des Transplantates nach erfolgter Transplantation zu treffen.

### Ausführung der Erfindung

Bevorzugte Ausführungsformen der Erfindung sind nachstehend angegeben:

Bevorzugt wird das erfindungsgemäße Verfahren zur Ermittlung der Leberfunktion auf der Basis von HepG2/C3A-Zellen durchgeführt.

Bei der HepG2/C3A-Zelllinie (ATCC #CRL10741) handelt es sich um einen Subklon der weit verbreiteten humanen Hepatoblastom-Zelllinie HepG2.

HepG2/C3A-Zellen sind gut differenziert. Sie besitzen viele phäno- und genotypische Eigenschaften der normalen Leberzellen und tragen keine viralen Sequenzen. In der Wachstumsphase vor dem Konfluieren verhalten sich die HepG2/C3A-Zellen ähnlich dem sich regenerierendem Lebergewebe. Sie haben eine kurze Verdopplungszeit von nur 24 Stunden und sezernieren eine Reihe fetaler Proteine, darunter unter anderem [alpha]-Fetoprotein, Aldolase A und C und Pyruvatkinase K.

Sobald die Zellen das Stadium der Konfluenz erreichen, kommt es zu einer starken Kontakthemmung. Die Häufigkeit der Zellteilungen verringert sich drastisch, so dass die Verdopplungszeit auf über 200 Stunden ansteigt und sich ein Gleichgewicht ausbildet. Es überwiegen nun HepG2/C3A-Zellen, die einen adulten Phänotyp darstellen. Dies wird auch an der Protein-Produktion deutlich: Die Bildung fetaler Proteine nimmt ab, statt dessen findet eine vermehrte Synthese von Albumin, Aldolase B und Pyruvatkinase L statt.

Außer zur Bildung verschiedener Plasmaproteine, wie zum Beispiel Albumin, Haptoglobin, Coeruloplasmin, [alpha]2-Makroglobulin, Transferrin, Plasminogen, Fibrinogen, sind HepG2/C3AZellen auch zur Harnstoffsynthese aus Lactat und Ammoniumchlorid in der Lage.

Ferner können die HepG2/C3A-Zellen in glucosefreiem Medium kultiviert werden. Dies zeigt, dass die besagten Zellen die Fähigkeit zur Gluconeogenese, welche eine der wichtigsten leberspezifischen Stoffwechselfunktionen ist, besitzen.

Die Aktivität von Cytochrom P450 1A2, einem an der Biotransformation verschiedener Arzneimittel beteiligten Enzym, ist bei HepG2/C3A-Zellen zwar gering, kann jedoch durch Induktion mit Methylcholanthren auf ein über 40faches der Ausgangsaktivität gesteigert werden (12).

In einer Versuchsreihe über den Mechanismus von Tumorinvasivität und Metastasierung wurden verschiedene humane Leberzellinien, unter anderem die Zelllinien C3A, HLE, HepG2, HuH7, Hep3B, PLC/PRP/5, untersucht. Die HepG2/C3A-Zellen zeigten hierbei die geringste Invasivität.

Aufgrund der weitgehenden leberspezifischen Differenzierung der HepG2/C3A-Zellinie wurden die Zellen als Bestandteil eines künstlichen Leberunterstützungssystems zur Therapie des Leberversagens bereits erprobt (10). Diese Leberzelllinien sind kommerziell erhältlich.

**Kultivierung der HepG2/C3A-Zellen:**

HepG2/C3A-Zellen werden in einem Brutschrank bei 37[deg.]C und mit 5 % CO₂-Begasung Gewebekulturflaschen mit 4 bis 20 ml Nährlösung (Medium) kultiviert. Diese besteht aus Dulbecco's Modified Eagle Medium (Life Technologies Ltd.) mit 10 % fetalem Kälberserum (=FCS, PAA), 1 % Glutamin-Lösung (200 mM, Serva) und 1 % Antibiotika-Lösung (Penicillin G (10.000 IE/ml) / Streptomycin (10 mg/ml) - Jenapharm ratiopharm GmbH). Zur Kryokonservierung werden HepG2/C3A-Zellen bei -196[deg.]C in flüssigem Stickstoff eingelagert in Tubes, die 1x10⁷ Zellen in 2 ml Medium mit 10% DMSO (Dimethylsulfoxid) enthalten. Eine Working-Zellbank wurde gebildet. Zur Revitalisierung werden die Zellen in oben beschriebenes Zellmedium überführt und nach Zellzählung mit Trypanblau-Färbung in der Neubauer-Zählkammer (siehe unten) auf eine Konzentration von 5 x 10⁵ Zellen / ml ausgesät. Der erste Mediumwechsel erfolgt nach 24 Stunden, danach drei Mal pro Woche. Die Revitalisierungszeit beträgt 14 Tage. Anschließend werden die Zellen alle 7 - 10 Tage gespurtet und in einer Konzentration von 5 x 10⁵ Zellen / ml neu ausgesät. Dabei wird eine maximale Splitzahl (Passagen) von 10 nicht überschritten.

**Bestimmung der Parameter**

1. Aktivitätsbestimmung von Cytochrome P450 Isoenzym 1A2 (Etoxyresorfin-Test, Aktivitätsbestimmung der Ethoxyresorussn-O-Deethylase, 11-12)

Die Aktivität von Cytochrom P450 1A2 lässt sich durch die O-Deethylierung von 7- Ethoxyresorufin zu Resorufin bestimmen.

Dazu werden HepG2/C3A-Zellen in 24-Well-Zellkulrurplatten für 3 Tage eingesät in einer Konzentration von 250.000 Zellen / Well und mit 0,5 ml Medium versetzt. Während dieser Zeit wird täglich ein Mediumwechsel durchgeführt. Um die CYP 1A2- Aktivität der Zellen zu erhöhen wird mit 3 -Methyl cholanthren (Sigma) in einer Konzentration von 5,36 g/l (20 µM) induziert. Vom vierten bis zum sechsten Tag werden die Zellen mit dem Probandenplasma (0,5 ml) inkubiert.

Die Aktivitätsmessung findet am sechsten Tag nach der Einsaat der Zellen statt. Zuerst wird das Zellkulturmedium / Probandenplasma abgesaugt, dann erhalten die Zellen 0,5 ml Medium mit Ethoxyresorufin (8 µM) und Dicumarol (10 µM) und müssen eine Stunde bei 37[deg.]C im Brutschrank inkubiert werden. Dabei wird ein Teil des Ethoxyresorufins von den HepG2/C3A-Zellen zu Resorufin umgewandelt. Das Dicumarol hat die Aufgabe, eine weitere Verstoffwechslung des Resorufins zu verhindern.

Anschließend werden aus jedem Well 2 x 75 µl für Doppelbestimmungen entnommen und in eine schwarze 96-Well-Platte umgefüllt. Dann werden 15 µl einer Lösung von β- Glucuronidase (15 Fishman units) und Arylsulfatase (120 Roy units) hinzugegeben, um eventuell gebildete Resorufin-Komplexe aufzuspalten.

Im Anschluss findet eine erneute Inkubation für drei Stunden bei 37[deg.]C und unter Schütteln statt.

Danach werden 200 µl Ethanol (99,9%) in jedes Well pipettiert. Nach weiteren 5 bis 10 Minuten kann der Resorufin-Gehalt der Proben am Fluoreszenz-Reader (Fluoroskan Ascent, Labsystems) bei einer Exzitation von 530 nm und einer Emission von 584 nm gemessen werden.

Um die Fluoreszenzen in Resorufin-Mengen umrechnen zu können, wird bei jedem Versuch eine Resorufin-Standardkurve angefertigt (Blank - 10 pmol - 20 pmol - 40 pmol - 80 pmol). Bei jedem Versuch wird eine reine Mediumkontrolle mit und ohne Zellen durchgeführt und von jeder Probe eine Doppelbestimmung.

Protokoll zur Zeitoptimierung des Etoxyresorfin-Tests (experimentelles Stadium):

Vom 8. bis zum 14. Tag der Revitalisierung der Zellen wird dem Medium 3- Methylcholanthren in einer Konzentration von 2,68 g/l (10 µM) hinzugefügt. Zusätzlich wird über die gesamte Revitalisierungphase (14 Tage) mit einer höheren FCS-Konzentration von 15% gearbeitet. Danach beträgt die Inkubationszeit der Zellen mit 3-Methylcholanthren in einer Konzentration von 5,36 g/l (20 µM) einen Tag. Die Inkubationszeit mit dem Probandenplasma beträgt nur noch einen Tag, so dass nach 48 Stunden nach Testbeginn ein Resultat vorliegt.

2. Syntheseleistung, z.B. Mikroalbuminund andere Proteinsynthesen der HepG2/C3AZellen (13)

Die HepG2/C3A-Zellen werden in 24-Well-Zellkulturplatten eingesät in einer Konzentration von 250.000 Zellen / Well und für 3 Tage mit 0,5 ml Probandenplasma + 0,25 ml Medium versetzt, danach einmal mit Medium gespült und anschließend mit frischem Medium (1 ml) für 3 Tage inkubiert. Im Anschluss wird aus 0,2 ml Zellkulturüberstand Mikroalbumin nephelometrisch als Doppelbestimmung (+ Mediumkontrolle) ermittelt. Jeder Versuchsansatz mit Probandenplasma wird ebenfalls als Doppelbestimmung durchgeführt.

Protokolle zur Optimierung der Proteinsynthese-Test (experimentelles Stadium):

Durch kontinuierliches Schütteln der Zellkulturflaschen durch einen industriell verfügbaren Schüttler wird der Zeil-Medium- bzw. Zell-Plasma-Kontakt optimiert. Dadurch sind die Inkubationszeiten sowohl mit dem Probandenplasma, als auch mit dem Medium auf einen Tag zu verkürzen, so dass nach 48 Stunden nach Testbeginn ein Resultat vorliegt.

Durch die Zugabe von Ornithin alpha-Ketoglutarate (=OKG, 0,03 mM, 13a) zum Medium wird die Syntheseleistung der HepG2/C3A-Zellen gesteigert. So kann die Testzeit auf 48 Stunden ebenfalls verkürzt werden und zweitens, setzen die Zellen durch die OKG Stimulation messbare Mengen an Fibrinogen und Transferrin (Plasmaproteine) frei, die in 200 µl Zellkulturüberstand als Doppelbestimmung gemessen werden.

3. Bestimmung der Freisetzung von Lactat-Dehydrogenase (LDH) aus den HepG2/C3AZellen (Vitalitätstest, 14):

Die HepG2/C3A-Zellen werden in 24-Well-Zellkulturplatten eingesät und für 3 Tage in einer Konzentration von 250.000 Zellen/Well mit 0,5 ml Probandenplasma + 0,25 ml Medium versetzt, danach einmal mit Medium gespült und anschließend mit frischem Medium (1 ml) für 3 Tage inkubiert. Im Anschluss wird aus 0,2 ml Zellkulturüberstand LDH als Doppelbestimmung ermittelt. Jeder Versuchsansatz mit Probandenplasma wird ebenfalls als Doppelbestimmung durchgeführt. Ebenfalls erfolgt eine Mediumkontrolle.

Die LDH-Bestimmung erfolgte nach der optimierten Standardmethode der Deutschen Gesellschaft für Klinische Chemie (DGKC). Dabei wird Pyruvat als Substrat verwendet und photometrisch die NADH-Abnahme bestimmt.

Protokolle zur Zeitoptimierung des LDHVitalitätstests (experimentelles Stadium):

Durch kontinuierliches Schütteln der Zellkulturflaschen durch einen industriell verfügbaren Schüttler wird der Zeil-Medium- bzw. Zell-Plasma-Kontakt optimiert. Dadurch sind die Inkubationszeiten sowohl mit dem Probandenplasma, als auch mit dem Medium auf einen Tag zu verkürzen, so dass nach 48 Stunden nach Testbeginn ein Resultat vorliegt.

**Weitere Vitalitätsund Proliferationstests:**

Die HepG2/C3A-Zellen werden für 3 Tage in 24-Well-Zellkulturplatten in einer Konzentration von 250.000 Zellen / Well mit 0,5 ml Probandenplasma + 0,25 ml Medium versetzt, danach einmal mit Medium gespült und anschließend mit frischem Medium (1 ml) für 3 Tage inkubiert. Eine Mediumkontrolle wird ebenfalls durchgeführt. Im Anschluss werden mit den Zellen folgende Teste durchgeführt:

4. Adherenz-Test

Zunächst wird semiquantitativ die Adherenz der HepG2/C3A-Zellen lichtmikroskopisch erfasst. Da es sich bei der HepG2/C3A-Zelllinie um eine adherente Ziellinie handelt, spricht ein hoher Anteil von adherenten Zellen im Well für eine gute Vitalität. Die Erfassung der Adherenz erfolgt als Doppelbestimmung.

5. Life/Dead-Test

Beim Life/Dead-Test (Molecular Probes, 15) werden die Zellen mit 2 Farbstoffen (Calcein AM und Ethidium Homodimer) versetzt.

CalceinAceton-Methylester (Calcein Green AM) kann aufgrund seiner Membrandurchlässigkeit in die vitale Zelle eindringen und wird intrazellulär durch zelleigene Esterasen gespalten (Hydroxylierung). Das entstehende Calcein bildet mit Calciumionen Chealte, die die Zelle nicht verlassen können. Die Calcein-Chelate emitieren bei einer Anregungswellenlänge von 485 nm Licht mit einer Wellenlänge von 530 nm. Es zeigt sich dann eine grünliche Fluoreszens, die mit einem Fluoesenzmikroskop aufgefangen und fotografiert werden kann. Bei toten Zellen ist die Esteraseaktivität aufgehoben, so dass sehr spezifisch nur vitale Zellen eine grünliche Fluoreszens zeigen.

Für tote Zellen hingegen ist der nicht membrangängige Zellfarbstoff Ethidiumbromidhomodimer geeignet. Dieser Farbstoff kann die intakten Zellmembranen nicht passieren, d.h. nicht durch die vitalen Zellen diffundieren, so dass dieser Farbstoff von vitalen Zellen nicht aufgenommen wird. In toten Zellen bildet der Farbstoff einen rötlich erscheinenden Fluoresenzkomplex mit Nukleinsäuren (Anregungsweilenlänge: 514 nm, emitierte Wellenlänge: 642 nm).

Zu Beginn des Testes wird das Medium vorsichtig abgesaugt. Dann werden 0,5 ml Live/Dead-Reagenz (Calcein AM: 2 µM, Ethidium Homodimer 4 µM) dazugeben. Danach erfolgt die Inkubation der Zellen im Brutschrank bei 37 °C unter Standartbedingungen für 30 Minuten. Im Anschluss werden die Zellen mit monochromatischem Licht fotografiert und der Test quantitativ ausgewertet. Dazu werden im Fluoreszenz-Reader (Fluoroskan Ascent, Labsystems) sowohl eine Grünmessung, als auch eine Rotmessung mit den oben genannten Wellenlängen durchgeführt. Es werden stets Doppelbestimmungen durchgeführt und ein Mittelwert daraus gebildet.

6. XTT-Test

Im XTT-Test (Roche, 16-17) wird die Umsetzung des Tetrazoliumsalzes und die dabei entstehende Farbänderung, die eine Veränderung der Extinktionswertes bewirkt, gemessen. Dabei entsteht durch metabolisch aktive Zellen aus dem gelben Tetrazoliumsalz XTT das orangefarbene Formazan. Durch mitochondriale Dehydrogenasen erfolgt die Umsetzung des gelben Tetrazoliumsalzes. Die Umsetzung des Tetrazoliumsalzes ist direkt proportional zum Aktivitätszustand der Zellen.

Zu Beginn des Testes wird das Medium vorsichtig abgesaugt. Dann werden 0,5 ml frisches Medium und 0,25 ml XTT-Reagenz in einer Konzentration von 1 mg/ml dazugeben. Danach erfolgt die Inkubation der Zellen im Brutschrank bei 37 °C unter Standartbedingungen. Nach 2, 4 und 24 Stunden erfolgen die Messungen der Extinktionen (Anthos Reader) als Doppelbestimmungen (Wellenlänge: 450 nm, Referenzwellenlänge: 690 nm). Dazu werden aus jedem Well 2 x 100 µl für Doppelbestimmungen entnommen und in eine 96-Well-Platte umgefüllt.

7. Trypanblau-Vitalitätstest

Die Zellen werden nach Trypsinierung mit Trypanblau angefärbt und in der Neubauerzählkammer die Anzahl der toten (blau leuchtend) / lebenden (nicht angefärbt) Zellen erfasst.

Zu Beginn des Testes wird das Medium vorsichtig aus dem Well abgesaugt, in 15 ml BlueCaps abgefüllt und die eventuell vorhandenen nicht adherenten Zellen abzentrifugiert. Das abzentrifugierte Medium wird verworfen. Die adherenten Zellen im Well werden mit 0,5 ml Trypsin-Lösung (1,25 mg/ml) und 1,5 ml Medium versetzt, um die Zellen abzulösen. Danach erfolgt die Inkubation bei 37[deg.]C unter Schütteln für 15 Minuten. Die gelösten Zellen werden anschließend mit dem Medium in das vorbereitete BlueCap gegeben, in dem sich die eventuell nicht adherenten Zellen befinden. Die Zellen werden resuspendiert und es erfolgt die Zellzahlbestimmung mit Trypanblau in der Neubauerzählkammer. Dazu werden 25 µl von der Zellsuspension mit 25 µl Trypanblau gemischt und danach in die Zählkammer gegeben. Es werden zwei Großquarte ausgezählt und das Ergebnis mit 10.000 multipliziert. So wird die Anzahl der lebenden, als auch der toten Zellen ermittelt. Alle Bestimmungen werden im Doppelansatz durchgeführt.

Protokolle zur Zeitoptimierung der Trypanblau-Vitalitätstests (experimentelles Stadium):

Durch kontinuierliches Schütteln der Zellkulturflaschen durch einen industriell verfügbaren Schüttler wird der Zeil-Medium- bzw. Zell-Plasma-Kontakt optimiert. Dadurch sind die Inkubationszeiten sowohl mit dem Probandenplasma, als auch mit dem Medium auf einen Tag zu verkürzen, so dass nach 48 Stunden nach Testbeginn ein Resultat vorliegt.

**Beispiele**

Der erfindungsgemäße Zytotoxizitätstest wurde erprobt bei Patienten mit Leberversagen um die Effektivität des Leberunterstützungsverfahrens MARS (Molecular Adsorbents Recirculation System, 18) zur Senkung endogener Toxine, die zur weiteren Leberschädigung führen, nachzuweisen. Dabei wurden die Zellen mit den Plasmaproben der Patienten vor und nach einer MARS Behandlung inkubiert. Mit Hilfe des Zytoxizitätstestes konnten deutliche Unterschiede zwischen den Patientenproben vor und nach einer MARS-Therapie und im Vergleich zu Proben von gesunden Probanden nachgewiesen werden, im Sinne einer besseren Funktion, Syntheseleistung und Vitalität der HepG2/C3A-Zellen, wenn sie mit dem Patientenplasma nach der MARS Therapie inkubiert wurden.

Der erfindungsgemäße Zytotoxizitätstest fand ferner Anwendung im Rahmen einer Sepsis-Therapie-Studie (EIS S -IStudie) mit dem extrakorporalen Plasmaperfusionmodell EISS (Extracorporeal Immune Support System, 19), der auf Spendergranulozyten basiert. In dieser Studie wurden Plasmaproben von Patienten mit septischen Schock im Verlauf der Therapie bestimmt (vier Abnahmezeitpunkte) und dazu im Vergleich Plasmaproben von gesunden Probanden gemessen. Dabei fielen deutliche Unterschiede zwischen den Plasmaproben von Patienten und gesunden Probanden, im Sinne einer eingeschränkten Vitalität, Syntheseleistung und Funktion der HepG2/C3A-Zellen bei den Patienten, auf. Markante Unterschiede traten auch bei den Ergebnissen der Patienten zwischen überlebenden und nicht-überlebenden HepG2/C3A-Zellen im dazu zeitlich stehenden Verlauf auf, so dass der entwickelte Zytoxizitätstest auch einen Stellenwert zur Therapieüberwachung und als Prognoseparameter besitzt.

Derzeit läuft eine große klinische Studie, bei welcher der Test an 50 Patienten mit schwerer Sepsis oder septischem Schock im Vergleich zu einem "nichtseptischem" Patientenkollektiv (50 Patienten) erprobt wird. Bei allen Patienten darf keine vorbestehende Lebererkrankung diagnostiziert sein.

Weiterhin wird der entwickelte Zytoxizitätstest derzeit im Rahmen einer Studie bei 15 Patienten validiert, die sich bei Leberversagen einer Lebertransplantation unterziehen müssen. Der Test soll dabei helfen, genauere Aussagen über die Funktion des Transplantates im weiteren Verlauf treffen zu können.

### Literatur

(1) Bakker J, Grover R, McLuckie A, Holzapfel L, Andersson J, Lodato R, Watson D, Grossman S, Donaldson J, Takala J; Glaxo Wellcome International Septic Shock Study Group: Administration of the nitric oxide synthase inhibitor NGmethyl-L-arginine hydrochloride (546C88) by intravenous infusion for up to 72 hours can promote the resolution of shock in patients with severe sepsis: results of a randomized, double-blind, placebocontrolled multicenter study (study no. 144-002). Crit Care Med 32 (1):1-12, 2004
(2) Harbrecht BG, Zenati MS5 Doyle HR, McMichael J, Townsend RN, Clancy KD, Peitzman AB: Hepatic dysfunction increases length of stay and risk of death after injury. J Trauma 53 (3): 517-23, 2002
(3) Kimura S, et al: Indocyanine green elimination rate detects hepatocellular dysfunction early in septic shock and correlates with survival. Crit Care Med 29: 1159-63, 2001
(4) Sakka S, et al: Prognostic Value of the Indocyanine Green Plasma Disappearance Rate in Critically 111 Patients. Chest 122: 1715-20, 2002
(5) Kobayashi M, Tsujitani S, Kurisu Y, Kaibara N: Bcl-2 and Bax expression for hepatocellular apoptosis in a murine endotoxin shock model. Hepatogastroenterology 49 (48): 1602-6, 2002
(6) Nöldge-Schomburg GFE, Priebe HJ, Armbruster K, Pannen B, Haberstroh J, Geiger K: Different effects of early endotoxaemia on hepatic and small intestinal oxygenation in pigs. Intensive Care Med 22: 795-804, 1996
(7) Rolando N, Wade J, Davalos M, Wendon J, Philpott-Howard J, Williams R: The Systemic Inflammatory Response Syndrom in Acute Liver Failure. Hepatology 32 (4): 734-9, 2000
(8) Angus DC, Linde-Zwirble WT, Lidicker J, Clermont G, Carcillo J, Pinsky MR: Epidemiology of severe sepsis in the United States: Analysis of incidence, o[mu]tcome and associated costs of care. Crit Care Med 29 (7): 1303-9, 2001
(9) Kelly JH, Darlington GJ: Modulation of the Liver Specific Phenotype in the Human Hepatoblastoma Line HEP G2. In Vitro Cellular and Developmental Biology 25 (2): 217-22, 1989
(10) Ellis AJ, Hughes RD, Wendon JA, et al: Pilot-controlled trial of the extracorporeal liver assist device in acute liver failure. Hepatology 24: 1446-51, 1996
(11) Kelly JH, Sussman NL: A Fluorescent Cell-Based Assay for Cytochrome P-450 Isozyme 1A2 Induction and Inhibition. Journal of Biomolecular Screening 5(4): 249-253, 2000
(12) Donato MT, G[omicron]mez-Lech[omicron]n MJ, Castell JV: A Microassay for Measuring Cytochxome P450IA1 and P450IIB1 Activities in Intact Human and Rat Hepatocytes Cultured on 96- Well Plates. Analytical Biochemistry 213: 29-33, 1993
(13) Krasteva N, Groth TH, Fey-Lambrecht F, Altankow G: The role of surface wettability on hepatocyte adhesive interactions and function. J Biomater 12 (6): 613-27, 2001
   (13a) Lescoat G, Desvergne B, Loreal O, Pasdeloup N, Deugnier Y, Bourel M, Brissot P: Modulation of Albumin Secretion by Ornithine Alpha-Ketoglutarate in Adult Rat Hepatocyte Culture and a Human Hepatoma Cell Line (HepG2). Ann Nutr Metab 33: 252-60, 1989
(14) Modrianski M, UMchova J, Bachleda P, Anzenbacher P, Anzenbacherova E, Walterova D, Simanek V: Human Hepatocyte - A Model for Toxicological Studies. Functional and Biochemical Characterization. Gen Phydiol Biophys 19: 223-35, 2000
(15) Dehn PF, White CM, Conners DE, Shipkey G, Cumbo TA: Characterization of the human hepatocellular Carcinoma (hepg2) cell line as an in vitro model for cadmium toxicity studies. In Vitro Cell Dev Biol Anim 40 (5-6): 172-82, 2004
(16) Scudiero DA, Shoemarker RH, Pauli KD, Monks A, Tierney S, Nofziger TH, Currens MJ5 Seniff D, Boyd MR: Evaluation of a soluble tetrazolium/formazan assay for cell growth and drug sensitivity in culture using human and other tumor cell lines. Cancer Res 48 (17): 4827-33, 1988
(17) Gressner AM, Polzar B, Lahme B, Mannherz H: Induction of rat liver parenchymal cell apoptosis by hepatic myofibroblasts via transforming growth factor ss. Hepatology 23 (3): 571-81, 2003
(18) Sen S, Mookerjee RP, Davies NA, Williams R, Jalan R: Review article: the molecular adsorbents recirculating System (MARS) in liver failure. Aliment Pharmacol Ther 16(5): 32- 8, 2002
(19) Mitzner SR, Freytag J, Sauer M, Kleinfeldt T, Altrichter J, Klöhr S, Koball S, Stange J, Ringel B, Nebe B, Schmidt H, Podbielski A, Noeldge-Schomburg G, Schmidt R: Use of human preconditioned phagocytes for extracorporeal immune support: introduction of a concept. Ther Apher 5 (5): 423-32, 2001

## Patentansprüche

1. Verwendung von humanen Hepatozyten zur in-vitro- Ermittlung der Leberfunktion bei einem Probanden, wobei eine Messung der Zellschädigung der humanen Hepatozyten nach Inkubation mit Probandenplasma erfolgt.

2. Verwendung nach Anspruch 1, wobei die humanen Hepatozyten aus den humanen, kommerziell erhältlichen, Leberzelllinien C3A, HLE, HepG2, HuH7, Hep3B, PLC/PRF/5 ausgewählt sind.

3. Verwendung nach Anspruch 1, wobei die humanen Hepatozyten HepG2/C3A-Zellen sind.

4. Verwendung nach Anspruch 1, 2 oder 3 zur Früherkennung eines Leberversagens, zur Überprüfung von Therapien, des Behandlungsverlaufes und der Regeneration der Leberfunktion und als Prognoseparameter.

5. Verwendung nach Anspruch 4, wobei der Proband an Systemic Inflammatory Response Syndrome (SIRS), Sepsis, schwere Sepsis, septischen Schock, Leberversagen oder Multiorganversagen erkrankt ist.

6. In-vitro-Verfahren zur Ermittlung der Leberfunktion bei einem Probanden unter Verwendung von humanen Hepatozyten, wobei nach Inkubation der humanen, kommerziell erhältlichen, Hepatozyten mit Probandenplasma, die Leberfunktion des Probanden mittels Messung der Parameter Vitalität, Funktionalität und Aktivierungsgrad der humanen Hepatozyten abgeleitet wird und die humanen Hepatozyten aus den humanen Leberzelllinien C3A, HLE, HepG2, HepG2/C3A-Zellen, HuH7, Hep3B, PLC/PRF/5 ausgewählt sind.

7. In-vitro-Verfahren Anspruch 6, wobei die humanen Hepatozyten HepG2/C3A-Zellen sind.

8. In-vitro-Verfahren nach einem der Ansprüche 6 oder 7, wobei die Leberfunktion des Probanden mittels Messung der Aktivität von Cytochrom P450 Isoenzym 1A2 (CYP 1A2) der humanen Hepatozyten abgeleitet wird.

9. in-vitro-Verfahren nach einem der Ansprüche 6 oder 7, wobei die Leberfunktion des Probanden mittels Messung der Aktivität der humanen Hepatozyten Mikroalbumin und andere Proteine zu synthetisieren abgeleitet wird.

10. In-vitro-Verfahren nach einem der Ansprüche 6 oder 7, wobei die Leberfunktion des Probanden mittels Messung der Freisetzung von Lactat-Dehydrogenase aus den humanen Hepatozyten abgeleitet wird.

11. In-vitro-Verfahren nach einem der Ansprüche 6 oder 7, wobei die Leberfunktion des Probanden mittels Messung der Adhärenz der vitalen humanen Hepatozyten abgeleitet wird.

12. In-vitro-Verfahren nach einem der Ansprüche 6 oder 7, wobei die Leberfunktion des Probanden mittels Messung der Esteraseaktivität der vitalen humanen Hepatozyten abgeleitet wird.

13. In-vitro-Verfahren nach einem der Ansprüche 6 oder 7, wobei die Leberfunktion des Probanden mittels Messung der Anzahl toter und der Anzahl lebender humaner Hepatozyten abgeleitet wird.

14. In-vitro-Verfahren nach einem der Ansprüche 6 oder 7, wobei die Leberfunktion des Probanden mittels Messung der Aktivität mitochondrialer Dehydrogenasen der vitalen humanen Hepatozyten abgeleitet wird.

## Claims

1. A use of human hepatocytes for in vitro investigation of the liver function on a subject wherein a measurement of the cellular damage of said human hepatocytes is carried out after incubation with subject plasma.

2. A use as claimed in claim 1 wherein said human hepatocytes are selected from the human liver cell lines C3A, HLE, HepG2, HuH7, Hep3B, PLC/PRF/5 being commercially available.

3. A use as claimed in claim 1 wherein said human hepatocytes are HepG2/C3A-cells.

4. A use as claimed in anyone of the claims 1, 2 or 3 for the early detection of a hepatic failure, for the verification of therapies, of the course of treatment and the regeneration of the liver function, and as a prognosis parameter.

5. A use as claimed in claim 4 wherein said subject fell ill with the systemic inflammatory response syndrome (SIRS), sepsis, severe sepsis, septic shock, liver failure, or multi organ failure.

6. An in vitro procedure for the investigation of the liver function on a subject using human hepatocytes wherein after incubation of said commercially available human hepatocytes with said subject plasma the liver function of said subject is deduced by means of measuring the parameters of vitality, functionality, and activation stage of said human hepatocytes, and said human hepatocytes are selected from the human liver cell lines C3A, HLE, HepG2, HepG2/C3A-cells, HuH7, Hep3B, PLC/PRF/5.

7. An in vitro procedure as claimed in claim 6 wherein said human hepatocytes are HepG2/C3A-cells.

8. An in vitro procedure as claimed in anyone of claim 6 or claim 7 wherein said liver function of said subject is deduced by means of measuring the activity of Cytochrome P450 isoenzyme 1A2 (CYP 1A2) of said human hepatocytes.

9. An in vitro procedure as claimed in anyone of claim 6 or claim 7 wherein said liver function of said subject is deduced by means of measuring the activity of said human hepatocytes to synthesize microalbumin and other proteins.

10. An in vitro procedure as claimed in anyone of claim 6 or claim 7 wherein said liver function of said subject is deduced by means of measuring the release of lactate dehydrogenase from said human hepatocytes.

11. An in vitro procedure as claimed in anyone of claim 6 or claim 7 wherein said liver function of said subject is deduced by means of measuring the adherence of the vital human hepatocytes.

12. An in vitro procedure as claimed in anyone of claim 6 or claim 7 wherein said liver function of said subject is deduced by means of measuring the esterase activity of said vital human hepatocytes.

13. An in vitro procedure as claimed in anyone of claim 6 or claim 7 wherein said liver function of said subject is deduced by means of measuring the number of dead human hepatocytes and the number of living human hepatocytes.

14. An in vitro procedure as claimed in anyone of claim 6 or claim 7 wherein said liver function of said subject is deduced by means of measuring the activity of mitochondrial dehydrogenases of said vital human hepatocytes.

## Revendications

1. Utilisation d'hépatocytes humains pour déterminer in vitro la fonction hépatique chez un sujet d'expérience, dans laquelle une mesure de l'endommagement cellulaire des hépatocytes humains a lieu après incubation avec du plasma du sujet d'expérience.

2. Utilisation selon la revendication 1, dans laquelle les hépatocytes humains sont choisis parmi les lignes de cellules hépatiques humaines commercialisées telles que C3A, HLE, HepG2, HuH7, Hep3B, PLC/PRF/5.

3. Utilisation selon la revendication 1, dans laquelle les hépatocytes humains sont des cellules HepG2/C3A.

4. Utilisation selon la revendication 1, 2 ou 3 pour la détection précoce d'une défaillance hépatique pour contrôler des thérapies, le déroulement du traitement et la régénération de la fonction hépatique et à titre de paramètre de pronostic.

5. Utilisation selon la revendication 4, dans laquelle le sujet d'expérience est affecté du syndrome de réponse inflammatoire systémique (SIRS), de septicémie, de septicémie grave, de choc septique, de défaillance hépatique ou de défaillance multiorganes.

6. Procédé in vitro pour déterminer la fonction hépatique d'un sujet d'expérience en utilisant des hépatocytes humains, dans lequel après l'incubation des hépatocytes humains commercialisés avec du plasma du sujet d'expérience, la fonction hépatique du sujet d'expérience est déduite au moyen de la mesure des paramètres tels que la vitalité, la fonctionnalité et le degré d'activation des hépatocytes humains, et les hépatocytes humains sont choisis parmi les lignes de cellules hépatiques humains telles que C3A, HLE, HepG2, les cellules HepG2/C3A, HuH7, Hep3B, PLC/PRF/5.

7. Procédé in vitro selon la revendication 6, dans lequel les hépatocytes humains sont des cellules HepG2/C3A.

8. Procédé in vitro selon l'une des revendications 6 ou 7, dans lequel la fonction hépatique du sujet d'expérience est déduite au moyen de la mesure de l'activité du cytochrome P450 et de l'isoenzyme 1 A2 (CYP 1 A2) des hépatocytes humains.

9. Procédé in vitro selon l'une des revendications 6 ou 7, dans lequel la fonction hépatique du sujet d'expérience est déduite au moyen de la mesure de l'activité des hépatocytes humains tels que la microalbumine et d'autres protéines à synthétiser.

10. Procédé in vitro selon l'une des revendications 6 ou 7, dans lequel la fonction hépatique du sujet d'expérience est déduite au moyen de la mesure du dégagement de lactate héshydrogénase à partir des hépatocytes humains.

11. Procédé in vitro selon l'une des revendications 6 ou 7, dans lequel la fonction hépatique du sujet d'expérience est déduite au moyen de la mesure de l'adhérence des hépatocytes humains vitaux.

12. Procédé in vitro selon l'une des revendications 6 ou 7, dans lequel la fonction hépatique du sujet d'expérience est déduite au moyen de la mesure des activités estérases des hépatocytes humains vitaux.

13. Procédé in vitro selon l'une des revendications 6 ou 7, dans lequel la fonction hépatique du sujet d'expérience est déduite au moyen de la mesure du nombre d'hépatocytes humains morts et du nombre d'hépatocytes humains vivants.

14. Procédé in vitro selon l'une des revendications 6 ou 7, dans lequel la fonction hépatique du sujet d'expérience est déduite au moyen de la mesure de déshydrogénases mitochondriale des hépatocytes humains vitaux.
